# EUROPEAN PATENT APPLICATION

(11) **EP 4 834 932 A1**
(43) Date of publication of application: **04.11.2026**
(21) Application number: 24910459.7
(22) Date of filing: 25.11.2024
(51) Int. Cl.: C07K 16/28, A61K 39/395, A61P 35/00

(54) **LY6G6D-TARGETED NANOBODY AND USE THEREOF**

(30) Priority: 26.12.2023 CN 202311797152
(71) Applicant: Sunshine Lake Pharma Co., Ltd., Dongguan, Guangdong 523000 (CN)
(72) Inventor: REN, Zhiheng, Dongguan, Guangdong 523871 (CN); LIN, Shushan, Dongguan, Guangdong 523871 (CN); LI, Lijia, Dongguan, Guangdong 523871 (CN); LI, Xiang, Dongguan, Guangdong 523871 (CN); CHEN, Miaojun, Dongguan, Guangdong 523871 (CN); XIAO, Lin, Dongguan, Guangdong 523871 (CN); WAN, Dan, Dongguan, Guangdong 523871 (CN); ZHANG, Kuo, Dongguan, Guangdong 523871 (CN); JIANG, Qiuyue, Dongguan, Guangdong 523871 (CN); ZHOU, Linjun, Dongguan, Guangdong 523871 (CN)
(74) Representative: Hoffmann Eitle
(86) International application number: PCT/CN2024/134172
(87) International publication number: WO 2025/139525

(57) **Abstract**

This invention provides a Ly6G6D-targeted nanobody and applications thereof. Specifically, the present invention provides a nanobody against Ly6G6D (lymphocyte antigen 6 complex, locus G6D) and antigen-binding fragments thereof, and further discloses the use of the aforementioned nanobody or antigen-binding fragments in the preparation of medicaments for treating Ly6G6D-positive cancers.

## Description

### FIELD OF THE INVENTION

The present invention belongs to the field of antibody engineering. Specifically, the present invention relates to a single-domain antibody, and in particular, to a nanobody targeting Ly6G6D and its use in the preparation of medicaments.

### BACKGROUND ART

Nanobodies are currently the smallest antibody molecules, initially discovered in camel blood by the Belgian scientist Hamers, and represent a highly regarded class of engineered antibody products. The main advantages of nanobodies are as follows: First, their small size, which is only one-tenth that of conventional antibodies. Due to their small size, they exhibit stronger penetration into animal tissues; for instance, they can penetrate human brain tissue and reach the interior of highdensity tumors, capabilities that conventional antibodies lack. This small-size advantage of nanobodies enables their use in treating certain tumors or brain diseases. Second, their stable efficacy; they adapt to a wide temperature range and remain functional at temperatures as high as 90°C, whereas conventional antibodies would denature under such conditions. They also exhibit remarkable stability even under extreme pH values, capable of passing through the human stomach while maintaining efficacy. Third, their excellent antigen specificity and ease of genetic modification. Nanobodies can recognize unique antigenic epitopes, possess broader antigen-binding capabilities than conventional antibodies, and are convenient for artificial modification to obtain antibodies against different pathogens. Furthermore, nanobodies can be easily synthesized in microorganisms and are amenable to large-scale expression in microbes such as bacteriophages, Escherichia coli and yeast, facilitating mass production.

Lymphocyte antigen 6 family member G6D (Ly6G6D) is a leukocyte antigen cluster located in the major histocompatibility complex (MHC) class III region on chromosome 6, encoding a protein of 133 amino acid residues with a molecular weight of approximately 13.7 kDa. Like most family members, Ly6G6D is attached to the cell membrane via a glycosylphosphatidylinositol (GPI) anchor. Multiple studies have demonstrated that lymphocyte antigen 6 family members are crucial for regulating immune, nervous system, and complement functions. Particularly in cancer, numerous lymphocyte antigen 6 family members play key roles in various cancer types, such as gastric cancer, cervical cancer, breast cancer, ovarian cancer, lung cancer, and bladder cancer. Therefore, the lymphocyte antigen 6 gene family is poised to play a significant role in clinical practice, serving not only as a prognostic marker for diseases but also as an important target for new drug development. In-depth investigation of their biological functions will be of great significance for elucidating the functions of the lymphocyte antigen 6 family, analyzing protein interactions within their structures, exploring disease pathogenesis, and discovering new therapeutic targets. As a new member of the lymphocyte antigen 6 gene family, Ly6G6D has been increasingly reported to play an important role in the development of diseases such as colorectal cancer. Currently, a bispecific antibody based on Ly6G6D is available for the treatment of colorectal cancer, indicating that Ly6G6D holds promise as a potential target for antibody-based therapies.

Existing ScFv, Fab, or full-length IgG class anti-Ly6G6D antibody molecules possess complex structures and large molecular sizes. Although they can conjugate active molecules to Ly6G6D, they impair the function of the active molecules, involve complex methodologies, and exhibit low loading efficiency. In contrast, nanobody molecules are small, easy to manipulate, and feature stable physicochemical properties and low immunogenicity.

### SUMMARY OF THE INVENTION

The present invention provides a Ly6G6D nanobody that exhibits high affinity for human recombinant Ly6G6D protein and possesses promising application prospects.

Specifically, in one aspect, the present invention provides a nanobody that specifically binds to Ly6G6D, wherein the nanobody is capable of specifically binding to Ly6G6D, and the heavy chain variable region of the nanobody comprises complementarity-determining regions: CDR1, CDR2 and CDR3;
wherein the CDR1 has the amino acid sequence shown in SEQ ID NO: 1 or SEQ ID NO: 2;
YYAIG (SEQ ID NO: 1);
SYX1MX2 (SEQ ID NO: 2); wherein X₁ is selected from A or M, and X₂ is selected from E or G;
the CDR2 has the amino acid sequence shown in SEQ ID NO: 3 or SEQ ID NO: 4;
CISSSGGSTVYADTVKG (SEQ ID NO: 3);
X₃X₄SWX₅GGX₆TYYADSX₇KG (SEQ ID NO: 4); wherein X₃ is selected from V or A; X₄ is selected from I, H or V; X₅ is selected from T or Y; X₆ is selected from V or S; and X₇ is selected from V or A;
the CDR3 has the amino acid sequence shown in SEQ ID NO: 5, SEQ ID NO: 6 or SEQ ID NO: 7;
GPECSRWYKDS (SEQ ID NO: 5);
ESRGFTHYNGNYYSRPDY (SEQ ID NO: 6);
RAPVKGDITPSNYYGLDY (SEQ ID NO: 7).

In some embodiments of the present invention, CDR1 has the amino acid sequence shown in SEQ ID NO: 1, SEQ ID NO: 8, SEQ ID NO: 9 or SEQ ID NO: 10;
CDR2 has the amino acid sequence shown in SEQ ID NO: 3, SEQ ID NO: 11, SEQ ID NO: 12 or SEQ ID NO: 13;
CDR3 has the amino acid sequence shown in SEQ ID NO: 5, SEQ ID NO: 6 or SEQ ID NO: 7.

In some embodiments of the present invention, CDR1 has the amino acid sequence shown in SEQ ID NO: 1; CDR2 has the amino acid sequence shown in SEQ ID NO: 3; CDR3 has the amino acid sequence shown in SEQ ID NO: 5.

In some embodiments of the present invention, CDR1 has the amino acid sequence shown in SEQ ID NO: 8; CDR2 has the amino acid sequence shown in SEQ ID NO: 11; CDR3 has the amino acid sequence shown in SEQ ID NO: 6.

In some embodiments of the present invention, CDR1 has the amino acid sequence shown in SEQ ID NO: 9; CDR2 has the amino acid sequence shown in SEQ ID NO: 12; CDR3 has the amino acid sequence shown in SEQ ID NO: 6.

In some embodiments of the present invention, CDR1 has the amino acid sequence shown in SEQ ID NO: 10; CDR2 has the amino acid sequence shown in SEQ ID NO: 13; CDR3 has the amino acid sequence shown in SEQ ID NO: 7.
YYAIG (SEQ ID NO: 1).
SYAME (SEQ ID NO: 8).
SYMME (SEQ ID NO: 9).
SYAMG (SEQ ID NO: 10).
CISSSGGSTVYADTVKG (SEQ ID NO: 3).
VISWTGGSTYYADSVKG (SEQ ID NO: 11).
VHSWTGGVTYYADSVKG (SEQ ID NO: 12).
AVSWYGGSTYYADSAKG (SEQ ID NO: 13).
GPECSRWYKDS (SEQ ID NO: 5).
ESRGFTHYNGNYYSRPDY (SEQ ID NO: 6).
RAPVKGDITPSNYYGLDY (SEQ ID NO: 7).

In some embodiments of the present invention, the heavy chain variable region further comprises framework regions: FR1, FR2, FR3 and FR4; wherein the framework regions and the complementarity-determining regions are arranged alternately in sequence.

Specifically, the alternating arrangement of the framework regions and the complementarity-determining regions is FR1-CDR1-FR2-CDR2-FR3-CDR3-FR4.

In some embodiments of the present invention, FR1 has the amino acid sequence shown in SEQ ID NO: 14;
QX₈QLVESGGGLVQX₉GGSLRLSCAASGX₁₀TX₁₁X₁₂ (SEQ ID NO: 14); wherein X₈ is selected from V or L; X₉ is selected from P or A; X₁₀ is selected from F or R; X₁₁ is selected from L or F; X₁₂ is selected from D, E or S;
FR2 has the amino acid sequence shown in SEQ ID NO: 15;
WFRQAPGKEREX₁₃VX₁₄ (SEQ ID NO: 15); wherein X₁₃ is selected from G or F; X₁₄ is selected from S or A;
FR3 has the amino acid sequence shown in SEQ ID NO: 16;
RFTISRDNX₁₅KNX₁₆ X₁₇YLQMX₁₈SLKPEDTAVYYCAA (SEQ ID NO: 16); wherein X₁₅ is selected from T or A; X₁₆ is selected from T or A; X₁₇ is selected from V or L; X₁₈ is selected from N or D;
FR4 has the amino acid sequence shown in SEQ ID NO: 17 or SEQ ID NO: 18;
WGQGTQVTVSS (SEQ ID NO: 17);
SGKGTLVIVSS (SEQ ID NO: 18).

In some embodiments of the present invention, FR1 has the amino acid sequence shown in SEQ ID NO: 19, SEQ ID NO: 20, SEQ ID NO: 21 or SEQ ID NO: 22;
FR2 has the amino acid sequence shown in SEQ ID NO: 23 or SEQ ID NO: 24;
FR3 has the amino acid sequence shown in SEQ ID NO: 25, SEQ ID NO: 26 or SEQ ID NO: 27;
FR4 has the amino acid sequence shown in SEQ ID NO: 17 or SEQ ID NO: 18.

In some embodiments of the present invention, FR1 has the amino acid sequence shown in SEQ ID NO: 19; FR2 has the amino acid sequence shown in SEQ ID NO: 23; FR3 has the amino acid sequence shown in SEQ ID NO: 25; and FR4 has the amino acid sequence shown in SEQ ID NO: 17.

In some embodiments of the present invention, FR1 has the amino acid sequence shown in SEQ ID NO: 20; FR2 has the amino acid sequence shown in SEQ ID NO: 24; FR3 has the amino acid sequence shown in SEQ ID NO: 26; and FR4 has the amino acid sequence shown in SEQ ID NO: 17.

In some embodiments of the present invention, FR1 has the amino acid sequence shown in SEQ ID NO: 21; FR2 has the amino acid sequence shown in SEQ ID NO: 24; FR3 has the amino acid sequence shown in SEQ ID NO: 26; and FR4 has the amino acid sequence shown in SEQ ID NO: 17.

In some embodiments of the present invention, FR1 has the amino acid sequence shown in SEQ ID NO: 22; FR2 has the amino acid sequence shown in SEQ ID NO: 24; FR3 has the amino acid sequence shown in SEQ ID NO: 27; and FR4 has the amino acid sequence shown in SEQ ID NO: 18.
QLQLVESGGGLVQPGGSLRLSCAASGFTLD (SEQ ID NO: 19).
QVQLVESGGGLVQAGGSLRLSCAASGRTFS (SEQ ID NO: 20).
QVQLVESGGGLVQAGGSLRLSCAASGRTFE (SEQ ID NO: 21).
QLQLVESGGGLVQAGGSLRLSCAASGRTFS (SEQ ID NO: 22).
WFRQAPGKEREGVS (SEQ ID NO: 23).
WFRQAPGKEREFVA (SEQ ID NO: 24).
RFTISRDNTKNTVYLQMDSLKPEDTAVYYCAA (SEQ ID NO: 25).
RFTISRDNAKNTVYLQMNSLKPEDTAVYYCAA (SEQ ID NO: 26).
RFTISRDNAKNALYLQMDSLKPEDTAVYYCAA (SEQ ID NO: 27).

In some embodiments of the present invention, the present invention provides a nanobody, wherein the heavy chain variable region of the nanobody has the amino acid sequence shown in any one of SEQ ID NO: 28 to 31, or an amino acid sequence having at least 85%, at least 90%, at least 95%, or at least 99% identity to any one of SEQ ID NO: 28 to 31.

In some embodiments of the present invention, the present invention provides a nanobody, wherein the heavy chain variable region of the nanobody has the amino acid sequence shown in any one of SEQ ID NO: 28 to 31, or an amino acid sequence having at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to any one of SEQ ID NO: 28 to 31.

QLQLVESGGGLVQPGGSLRLSCAASGFTLDYYAIGWFRQAPGKEREGVSCISSSGGSTV
YADTVKGRFTISRDNTKNTVYLQMDSLKPEDTAVYYCAAGPECSRWYKDSWGQGTQVTV

SS (SEQ ID NO: 28).

In the present invention, the antibody comprising the heavy chain variable region shown in SEQ ID NO: 28 is designated as:1A4.

QVQLVESGGGLVQAGGSLRLSCAASGRTFSSYAMEWFRQAPGKEREFVAVISWTGGST
YYADSVKGRFTISRDNAKNTVYLQMNSLKPEDTAVYYCAAESRGFTHYNGNYYSRPDYW
GQGTQVTVSS (SEQ ID NO: 29).

In the present invention, the antibody comprising the heavy chain variable region shown in SEQ ID NO: 29 is designated as:1D5.

QVQLVESGGGLVQAGGSLRLSCAASGRTFESYMMEWFRQAPGKEREFVAVHSWTGGV
TYYADSVKGRFTISRDNAKNTVYLQMNSLKPEDTAVYYCAAESRGFTHYNGNYYSRPDY
WGQGTQVTVSS (SEQ ID NO: 30).

In the present invention, the antibody comprising the heavy chain variable region shown in SEQ ID NO: 30 is designated as: 1D5G8.

QLQLVESGGGLVQAGGSLRLSCAASGRTFSSYAMGWFRQAPGKEREFVAAVSWYGGST
YYADSAKGRFTISRDNAKNALYLQMDSLKPEDTAVYYCAARAPVKGDITPSNYYGLDYSG
KGTLVIVSS (SEQ ID NO: 31).

In the present invention, the antibody comprising the heavy chain variable region shown in SEQ ID NO: 31 is designated as 2G5.

In some embodiments of the present invention, the nanobody described herein is alpaca-derived.

In another aspect, the present invention provides a fusion protein, wherein the fusion protein comprises a functional domain capable of specifically binding to Ly6G6D; wherein the functional domain is constituted by the nanobody described herein that specifically binds to Ly6G6D.

In some embodiments of the present invention, the fusion protein described herein further comprises a constant region.

Specifically, the constant region is the constant region of human IgG or a mutant thereof.

Specifically, the constant region of human IgG is the constant region of human IgG1 or a mutant thereof.

In some embodiments of the present invention, the constant region of IgG1 described herein comprises an Fc fragment, wherein the Fc fragment comprises the amino acid sequence shown in SEQ ID NO: 32.

EPKSADKTHTCPPCPAPELLGGPSVFLFPPKPKDTLMISRTPEVTCVVVDVSHEDPEVK

FNWYVDGVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPENNYKTTP
PVLDSDGSFFLYSKLTVDKSRWQQGNVFSCSVMHEALHNHYTQKSLSLSPGK (SEQ ID NO:
32).

In some embodiments of the present invention, the fusion protein described herein further comprises a linker peptide.

Specifically, the linker peptide of the fusion protein is used to connect the heavy chain variable region and the constant region, wherein the C-terminus of the heavy chain variable region is connected to the N-terminus of the constant region via the linker peptide. An example of the amino acid sequence of the linker peptide is: (GnS)m, wherein n is a natural number from 3 to 5, preferably 4, and m is a natural number from 1 to 5, preferably 3.
GGGGSGGGGSGGGGS (SEQ ID NO: 33).

In yet another aspect, the present invention provides an anti-Ly6G6D antibody, wherein the antibody is a conventional antibody or a functional fragment thereof, and the heavy chain variable region of the conventional antibody or functional fragment thereof is constituted by the nanobody described herein that specifically binds to Ly6G6D.

In some embodiments of the present invention, the functional fragment of the antibody is a Fab, Fab', (Fab')2, Fv, scFv or sdFv structure of a conventional antibody.

Conventional antibodies structurally consist of two identical heavy chains and two identical light chains. The light chain comprises a light chain variable region (VL) and a light chain constant region (CL); the heavy chain comprises a heavy chain variable region (VH) and heavy chain constant regions (CH1, CH2, CH3 and/or CH4). Given that the present invention discloses the structure of nanobodies capable of specifically binding to Ly6G6D, those skilled in the art would readily conceive of modifying conventional antibodies using the nanobodies of the present invention. For example, applying the CDR region structure of the nanobodies of the present invention to conventional antibodies can yield conventional antibodies capable of specifically binding to Ly6G6D. Such conventional antibodies also fall within the scope of protection of the present invention. Furthermore, based on the structure of conventional antibodies, partial structures thereof, such as Fab, Fab', (Fab')2, Fv, scFv, or sdFv, also possess Ly6G6D binding specificity and likewise fall within the scope of protection of the present invention.

Among these, Fab consists of a complete light chain along with the variable domain (VH) of the heavy chain and the first constant domain (CH1) of a heavy chain; Fab can be a product generated by papain digestion of an antibody. Fab' differs from Fab in that the Fab' fragment has several residues added to the carboxyl terminus of the CH1 domain, wherein the residues include one or more cysteines derived from the antibody hinge region. (Fab')2 corresponds to two Fab' fragments linked via disulfide bonds, possessing bivalent antigen-binding activity and remaining capable of crosslinking antigens; (Fab')2 can be a product of pepsin cleavage of an antibody. Fv consists of a tight, non-covalently associated dimer comprising one heavy chain variable domain and one light chain variable domain. scFv (single-chain antibody) refers to an antibody fragment containing VH and VL antibody domains linked into a single polypeptide chain; preferably, the scFv further comprises a polypeptide linker between the VH and VL domains, enabling the scFv to form the required antigen-binding structure. sdFv refers to an Fv fragment linked via disulfide bonds.

In yet another aspect, the present invention provides a bispecific or multispecific antibody comprising the nanobody of the present invention that specifically binds to Ly6 G6 D. To produce the bispecific or multispecific antibody, the nanobody of the present invention may be linked (e.g., via chemical conjugation, gene fusion, non-covalent association, or other means) to one or more other binding molecules (e.g., additional antibodies, antibody fragments, peptides, or binding mimetics).

In some embodiments, the bispecific or multispecific antibody specifically binds Ly6G6D and additionally specifically binds one or more other targets.

In some embodiments, the bispecific or multispecific antibody further comprises at least one second antibody having a second binding specificity against a second target.

In yet another aspect, the present invention also provides a conjugate comprising the nanobody or bispecific or multispecific antibody of the present invention that specifically binds L y6G6D, and a conjugation moiety.

In some embodiments, the nanobody of the present invention that specifically binds Ly6G6D is optionally conjugated to the conjugation moiety via a linker.

In some embodiments, the conjugation moiety is selected from protein tags. Such protein tags are well known in the art, and examples include but are not limited to His, Flag, GST, MBP, HA, Myc, GFP or biotin. Those skilled in the art know how to select an appropriate protein tag according to the desired purpose (e.g., purification, detection, or tracing).

In some embodiments, a His tag (e.g., 6×His) is linked to the C-terminus of the nanobody of the present invention that specifically binds Ly6G6D.

In some embodiments, the conjugation moiety is selected from detectable labels, such as enzymes (e.g., horseradish peroxidase), radionuclides, fluorescent dyes, luminescent substances (e.g., chemiluminescent substances), or biotin. The detectable label of the present invention may be any substance detectable by fluorescence, spectroscopic, photochemical, biochemical, immunological, electrical, optical, or chemical means. Such labels are well known in the art, and examples include but are not limited to enzymes (e.g., horseradish peroxidase, alkaline phosphatase, β-galactosidase, urease, glucose oxidase, etc.), radionuclides (e.g., ³H, ¹²⁵I, ³⁵S, ¹⁴C, or ³²P), fluorescent dyes (e.g., fluorescein isothiocyanate (FITC), fluorescein, tetramethylrhodamine isothiocyanate (TRITC), phycoerythrin (PE), Texas Red, rhodamine, quantum dots, or cyanine dye derivatives (e.g., Cy7, Alexa 750)), luminescent substances (e.g., chemiluminescent substances such as acridinium ester compounds), magnetic beads (e.g., Dynabeads^{®}), calorimetric labels such as colloidal gold or colored glass or plastic (e.g., polystyrene, polypropylene, latex, etc.) beads, and biotin for binding avidin (e.g., streptavidin) modified with the aforementioned labels.

In some embodiments, such labels are suitable for immunological assays (e.g., enzyme-linked immunosorbent assay, radioimmunoassay, fluorescent immunoassay, chemiluminescent immunoassay, etc.).

In some embodiments, the detectable labels described above can be conjugated to the nanobody of the present invention or its antigen-binding fragment via linkers of different lengths to reduce potential steric hindrance.

In some embodiments, the conjugated moiety is selected from therapeutic agents, such as antiinflammatory drugs or immunosuppressants.

In some embodiments, the conjugated moiety is selected from additional bioactive polypeptides.

In yet another aspect, the present invention provides an isolated nucleic acid molecule comprising a nucleotide sequence encoding the nanobody of the present invention, the antibody of the present invention, or the fusion protein of the present invention.

In some embodiments of the present invention, the nucleic acid molecule is DNA.

In some embodiments of the present invention, the present invention further provides a nucleic acid construct comprising the nucleic acid molecule of the present invention; further, the nucleic acid construct comprises one or more control sequences operably linked to the nucleic acid molecule, wherein the one or more control sequences direct the production of the antibody of the present invention or its antigen-binding fragment in a suitable host cell, and the one or more control sequences are selected from: promoters, enhancers, stop signals, signal peptides, leader sequences, transcription terminators, and any combinations thereof.

In yet another aspect, the present invention provides an expression vector carrying the nucleic acid molecule of the present invention.

In some embodiments of the present invention, the expression vector of the present invention is selected from: plasmids, cosmids, viruses, minichromosomes and artificial chromosomes.

In some embodiments of the present invention, the expression vector is a eukaryotic expression vector.

In some embodiments of the present invention, the eukaryotic expression vector is selected from: pCRII, pCR3, pcDNA3.4, pBSII, pET15, pGEX, pEGFP-N1, pETL, pDSR-α, pFastBacDual, and the like.

In yet another aspect, the present invention provides a host cell comprising the nucleic acid molecule of the present invention or the expression vector of the present invention.

In some embodiments of the present invention, the host cell is Escherichia coli, yeast or a eukaryotic cell.

In some embodiments of the present invention, the host cell is a eukaryotic host cell.

In some embodiments of the present invention, the host cell is a mammalian host cell.

In another aspect, the present invention provides a pharmaceutical composition comprising the nanobody of the present invention, the antibody of the present invention, the fusion protein of the present invention, the nucleic acid molecule of the present invention, the expression vector of the present invention, or the host cell of the present invention.

In some embodiments of the present invention, the pharmaceutical composition of the present invention further comprises a pharmaceutically acceptable carrier.

The pharmaceutical composition of the present invention may include a "therapeutically effective amount" or a "prophylactically effective amount" of the nanobody of the present invention, the antibody of the present invention, the fusion protein of the present invention, the nucleic acid molecule of the present invention, the expression vector of the present invention, or the host cell of the present invention.

The term "prophylactically effective amount" refers to an amount sufficient to prevent, inhibit, or delay the onset of a disease. The term "therapeutically effective amount" refers to an amount sufficient to cure or at least partially arrest the disease and its complications in a patient already suffering from the disease. The therapeutically effective amount may vary depending on factors such as the severity of the disease to be treated, the general state of the patient's own immune system, the patient's general condition (e.g., age, weight, and sex), the mode of administration of the drug, and other concurrent treatments, among others.

The pharmaceutical composition of the present invention may be formulated into any dosage form known in the medical field, such as tablets, pills, suspensions, emulsions, solutions, gels, capsules, powders, granules, elixirs, lozenges, suppositories, injections (including injectable solutions, sterile powders for injection, and concentrated solutions for injection), inhalants, sprays, etc. The preferred dosage form depends on the intended route of administration and therapeutic use.

A preferred dosage form is an injection. Such injections may be sterile injectable solutions. For example, a sterile injectable solution may be prepared by incorporating the required dose of the antibody of the present invention or its antigen-binding fragment, and optionally other desired ingredients (including but not limited to pH adjusters, surfactants, adjuvants, ionic strength enhancers, isotonic agents, preservatives, diluents, or any combination thereof) into a suitable solvent, followed by sterilization by filtration. In addition, the sterile injectable solution may be prepared as a sterile lyophilized powder (e.g., by vacuum drying or freeze-drying) to facilitate storage and use.

The nanobody of the present invention, the antibody of the present invention, the fusion protein of the present invention, the nucleic acid molecule of the present invention, the expression vector of the present invention, the host cell of the present invention, or the composition of the present invention can be administered by any suitable method known in the art, including but not limited to oral, buccal, sublingual, ocular, topical, parenteral, rectal, intrathecal, intracisternal, intravaginal, intravesical, topical (e.g., powder, ointment, or drops), or nasal routes. However, for many therapeutic uses, the preferred route/mode of administration is parenteral administration (e.g., intravenous injection or bolus, subcutaneous injection, intraperitoneal injection, intramuscular injection). Those skilled in the art will understand that the route and/or mode of administration will vary depending on the intended purpose.

In some embodiments, the nanobody of the present invention, the antibody of the present invention, the fusion protein of the present invention, the nucleic acid molecule of the present invention, the expression vector of the present invention, the host cell of the present invention, or the composition of the present invention is administered via intravenous injection or bolus.

In yet another aspect, the present invention provides the use of the nanobody of the present invention, the antibody of the present invention, the fusion protein of the present invention, the nucleic acid molecule of the present invention, the expression vector of the present invention, the host cell of the present invention, or the pharmaceutical composition of the present invention in the manufacture of a medicament for treating or alleviating Ly6G6D-positive cancer in a subject in need thereof.

In some embodiments of the present invention, the Ly6G6D-positive cancer is colorectal cancer, head and neck cancer, ovarian cancer, cervical cancer, melanoma, gastric cancer, esophageal cancer, small intestine cancer, large intestine cancer or adenocarcinoma, etc.

In yet another aspect, the present invention provides a kit for detecting Ly6G6D, wherein the kit comprises the nanobody of the present invention, the antibody of the present invention, or the fusion protein of the present invention, and instructions for use.

In yet another aspect, the present invention provides a method for detecting the presence or amount of L y6G6D in a sample, comprising using the nanobody of the present invention.

In some embodiments, the method is an immunological assay, such as immunoblotting, enzyme immunoassay (e.g., ELISA), chemiluminescent immunoassay, fluorescent immunoassay or radioimmunoassay.

In some embodiments, the conjugate used in the method comprises a nanobody of the present invention and a detectable label.

In some embodiments, the nanobody used in the method is conjugated with a detectable label.

In some embodiments, the nanobody used in the method is not conjugated with a detectable label. Accordingly, the method may further comprise using other reagents bearing a detectable label (e.g., a secondary antibody) to detect the nanobody of the present invention.

In some embodiments, the method comprises the following steps:
(1) contacting the sample with the nanobody of the present invention;
(2) detecting the formation of a complex between the nanobody and Ly6G6D, or detecting the amount of the complex.

The formation of the complex indicates the presence of Ly6G6D or cells expressing Ly6G6D.

The method may be used for diagnostic purposes or non-diagnostic purposes (e.g., wherein the sample is a cell sample rather than a sample derived from a patient).

In some embodiments, the method is used to diagnose whether a subject suffers from a disease associated with Ly6G6D. In such embodiments, the method may further comprise the step of comparing the amount of Ly6G6D in a sample from the subject to a reference value. The reference value may be the level of Ly6G6D in a sample from a subject known not to have a disease associated with Ly6G6D (e.g., a healthy control), also referred to as a "negative reference value." For example, an elevated amount of Ly6G6D in the sample from the subject relative to the negative reference value indicates that the subject suffers from a disease associated with Ly6G6D.

In some embodiments, the sample may be selected from urine, blood, serum, plasma, saliva, ascites, circulating cells, circulating tumor cells, non-tissue-associated cells (i.e., free cells), tissues (e.g., surgically resected tumor tissue, biopsy tissue, or fine-needle aspiration tissue), histological preparations, and the like.

### DEFINITION OF TERMS

In the present invention, unless otherwise specified, scientific and technical terms used herein shall have the meanings commonly understood by those skilled in the art. Furthermore, the operational procedures for cell culture, molecular biology, biochemistry, nucleic acid chemistry, immunology, and the like used herein are conventional steps widely employed in the respective fields. Meanwhile, to facilitate a better understanding of the present invention, definitions and explanations of relevant terms are provided below.

When the present invention uses the terms "for example", "such as", "including", "comprising", or variants thereof, these terms are not to be considered limiting and shall be interpreted to mean "including but not limited to" or "not limited to".

Unless otherwise indicated herein or clearly contradicted by context, the terms "a" and "an" and "the" and similar referents in the context of describing the invention (especially in the context of the following claims) are to be construed to cover both the singular and the plural.

As used herein, the term "camelid-derived antibody" refers to an antibody produced against an antigen by an immunized or antigen-challenged animal of the family Camelidae (including Camel, Alpaca, and L. glama). It is known to those skilled in the art that among antibodies produced by camelids, there exist "heavy chain antibodies" (Camelid heavy chain antibody, HCAb) lacking light chains. Such antibodies comprise only one variable domain of the heavy chain (variable domain of heavy chain of HCAb, VHH) and two conventional CH2 and CH3 regions. Furthermore, the VHH region, when cloned and expressed individually, exhibits excellent structural stability and antigen-binding activity. VHH is currently known as the smallest unit capable of binding to a target antigen.

As used herein, the term "nanobody" has the meaning commonly understood by those skilled in the art, referring to an antibody fragment composed of a single monomeric variable antibody domain (e.g., a single heavy chain variable domain), typically derived from the variable region of a heavy chain antibody (e.g., a camelid antibody or a shark antibody). Typically, a nanobody consists of four framework regions and three complementarity-determining regions, having the structure FR1-CDR1-FR2-CDR2-FR3-CDR3-FR4. A nanobody may be truncated at the N-terminus or C-terminus to contain only partial FR1 and/or FR4, or to lack one or both of those framework regions, provided that it substantially retains antigen binding and specificity. Nanobodies are also referred to as single domain antibodies (sdAb), and the two terms are used interchangeably.

As used in the present invention, the term "antigen-binding fragment" of a nanobody refers to a polypeptide comprising a fragment of the nanobody that retains the ability to specifically bind the same antigen to which the nanobody binds, and/or to compete with the nanobody for specific binding to the antigen; it is also referred to as an "antigen-binding portion." See generally, Fundamental Immunology, Ch. 7 (Paul, W., ed., 2nd ed., Raven Press, N.Y. (1989)), which is incorporated herein by reference in its entirety for all purposes. Antigen-binding fragments of the antibodies of the present invention can be produced by recombinant DNA techniques or by enzymatic or chemical cleavage of the nanobodies of the present invention. In some embodiments, the "antigen-binding fragment" of the nanobody may be truncated at the N-terminus or C-terminus compared to the full-length nanobody such that it comprises only part of FR1 and/or FR4, or lacks one or both of those framework regions, provided that it substantially retains antigen binding and specificity.

Antigen-binding fragments of a nanobody can be obtained from a given nanobody (e.g., a nanobody provided by the present invention) using conventional techniques known to those skilled in the art (e.g., recombinant DNA techniques or enzymatic or chemical cleavage methods), and the antigen-binding fragments of the nanobody can be screened for specificity in the same manner as used for the intact nanobody.

Herein, unless the context clearly indicates otherwise, when referring to the term "nanobody," it includes not only the intact nanobody but also antigen-binding fragments of the nanobody.

As used in the present invention, the term "complementarity-determining region" or "CDR" refers to amino acid residues in the antibody variable region responsible for antigen binding. A nanobody contains three CDRs, designated CDR1, CDR2, and CDR3. The precise boundaries of these CDRs can be defined according to various numbering systems known in the art, for example, as defined in the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al., Dev. Comparat. Immunol. 27:55-77, 2003). For a given nanobody, those skilled in the art will readily identify the CDRs defined by each numbering system. Furthermore, the correspondence between different numbering systems is well known to those skilled in the art (see, e.g., Lefranc et al., Dev. Comparat. Immunol. 27:55-77, 2003).

As used in the present invention, the term "framework region" or "FR" residues refers to those amino acid residues in the antibody variable region other than the CDR residues as defined above.

As used in the present invention, the term "specific binding" refers to a non-random binding reaction between two molecules, such as the reaction between an antibody and its target antigen. The strength or affinity of a specific binding interaction can be expressed by the equilibrium dissociation constant (KD) of the interaction. In the present invention, the term "KD" refers to the equilibrium dissociation constant of a specific antibody-antigen interaction, which is used to describe the binding affinity between the antibody and the antigen. The smaller the equilibrium dissociation constant, the tighter the antibody-antigen binding and the higher the affinity between the antibody and the antigen.

The specific binding properties between two molecules can be determined using methods known in the art. One method involves measuring the rates of antigen binding site/antigen complex formation and dissociation. Both the "association rate constant" (ka or kon) and the "dissociation rate constant" (kdis or koff) can be calculated from the concentration and the actual rates of association and dissociation (see Malmqvist M, Nature, 1993, 361:186-187). The ratio of kdis/kon equals the dissociation constant KD (see Davies et al., Annual Rev Biochem, 1990; 59:439-473). The values of KD, kon, and kdis can be measured by any effective method. In certain embodiments, the dissociation constant can be measured in a Biacore using surface plasmon resonance (SPR). In addition, the dissociation constant can be measured using biolayer interferometry or Kinexa.

The term "amino acid" refers to a compound containing both amino and carboxyl functional groups, such as an α-amino acid. Two or more amino acids can form a polypeptide via amide bonds (also known as peptide bonds). A single amino acid is encoded by a nucleic acid composed of three nucleotides (so-called codons or base triplets). Each amino acid is encoded by at least one codon. The encoding of the same amino acid by different codons is referred to as "degeneracy of the genetic code". Amino acids include natural amino acids and non-natural amino acids. Natural amino acids include alanine (three-letter code: Ala, one-letter code: A), arginine (Arg, R), asparagine (Asn, N), aspartic acid (Asp, D), cysteine (Cys, C), glutamine (Gln, Q), glutamic acid (Glu, E), glycine (Gly, G), histidine (His, H), isoleucine (Ile, I), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y), and valine (Val, V).

As used in the present invention, the term "expression vector" refers to a nucleic acid vehicle into which a polynucleotide can be inserted. When a vector enables the expression of a protein encoded by the inserted polynucleotide, the vector is termed an expression vector. Vectors can be introduced into host cells via transformation, transduction, or transfection, enabling the genetic elements they carry to be expressed within the host cells. Vectors are well known to those skilled in the art and include, but are not limited to: plasmids; phagemids; cosmids; artificial chromosomes, such as Yeast Artificial Chromosomes (YAC), Bacterial Artificial Chromosomes (BAC), or P1-derived Artificial Chromosomes (PAC); bacteriophages such as λ phage or M13 phage; and animal viruses. Animal viruses that can be used as vectors include, but are not limited to, retroviruses (including lentiviruses), adenoviruses, adeno-associated viruses, herpesviruses (such as herpes simplex virus), poxviruses, baculoviruses, papillomaviruses, and papovaviruses (such as SV40). A vector may contain multiple elements controlling expression, including but not limited to promoter sequences, transcription initiation sequences, enhancer sequences, selection elements, and reporter genes. Additionally, vectors may also contain origins of replication.

As used in the present invention, the term"nucleic acid molecule" is intended to include DNA molecules and RNA molecules. Nucleic acid molecules may be single-stranded or double-stranded and may be cDNA.

As used in the present invention, the term "host cell" refers to a cell capable of receiving a vector, including but not limited to prokaryotic cells such as Escherichia coli or Bacillus subtilis; fungal cells such as yeast cells or Aspergillus species; insect cells such as Drosophila S2 cells or Sf9 cells; or animal cells such as fibroblasts, CHO cells, COS cells, NSO cells, HeLa cells, BHK cells, HEK 293 cells, or human cells. Host cells may comprise single cells or cell populations.

As used in the present invention, the term "identity" refers to the degree of sequence match between two polypeptides or between two nucleic acids. When a position in both sequences being compared is occupied by the same base or amino acid monomeric subunit (for example, when a position in each of two DNA molecules is occupied by adenine, or a position in each of two polypeptides is occupied by lysine), then the molecules are identical at that position. The "percent identity" between two sequences is a function of the number of matching positions shared by the two sequences divided by the number of positions compared, multiplied by 100. For example, if 6 out of 10 positions in two sequences match, then the two sequences have 60% identity. Typically, comparisons are made when two sequences are aligned to produce maximum identity. Such alignment can be achieved using, for example, the method of Needleman et al. (1970) J. Mol. Biol. 48:443-453, which can be conveniently performed via computer programs such as the Align program (DNAstar, Inc.). The percent identity between two amino acid sequences can also be determined using the algorithm of E. Meyers and W. Miller (Comput. Appl. Biosci., 4:11-17 (1988)) incorporated into the ALIGN program (version 2.0), using a PAM120 weight residue table, a gap length penalty of 12, and a gap penalty of 4. Furthermore, the percent identity between two amino acid sequences can be determined using the Needleman and Wunsch (J. Mol. Biol. 48:444-453 (1970)) algorithm in the GAP program incorporated into the GCG software package (available at www.gcg.com), using a BLOSSUM 62 matrix or a PAM250 matrix, and a gap weight of 16, 14, 12, 10, 8, 6, or 4, and a length weight of 1, 2, 3, 4, 5, or 6.

As used in the present invention, the term "pharmaceutically acceptable carrier" refers to carriers and/or excipients that are pharmacologically and/or physiologically compatible with the subject and the active ingredient, which are well known in the art (see, e.g., Remington's Pharmaceutical Sciences, Edited by Gennaro AR, 19th ed., Pennsylvania: Mack Publishing Company, 1995), and include but are not limited to: pH adjusters, surfactants, adjuvants, ionic strength enhancers, diluents, agents for maintaining osmotic pressure, agents for delaying absorption, and preservatives. For example, pH adjusters include but are not limited to phosphate buffers. Surfactants include but are not limited to cationic, anionic, or nonionic surfactants, such as Tween-80. Ionic strength enhancers include but are not limited to sodium chloride. Preservatives include but are not limited to various antibacterial and antifungal agents, such as parabens, chlorobutanol, phenol, sorbic acid, etc. Agents for maintaining osmotic pressure include but are not limited to sugars, NaCl, and analogs thereof. Agents for delaying absorption include but are not limited to monostearates and gelatin. Diluents include but are not limited to water, aqueous buffers (such as buffered saline), alcohols, and polyols (such as glycerol), etc. Preservatives include but are not limited to various antibacterial and antifungal agents, such as thimerosal, 2-phenoxyethanol, parabens, chlorobutanol, phenol, sorbic acid, etc. Stabilizers have the meaning commonly understood by those skilled in the art; they are capable of stabilizing the desired activity of the active ingredient in the drug, including but not limited to monosodium glutamate, gelatin, SPGA, sugars (such as sorbitol, mannitol, starch, sucrose, lactose, dextran, or glucose), amino acids (such as glutamic acid, glycine), proteins (such as dried whey, albumin, or casein), or their degradation products (such as lactalbumin hydrolysate), etc. In certain exemplary embodiments, the pharmaceutically acceptable carrier or excipient comprises a sterile injectable liquid (such as an aqueous or non-aqueous suspension or solution). In some exemplary embodiments, such sterile injectable liquids are selected from Water for Injection (WFI), Bacteriostatic Water for Injection (BWFI), sodium chloride solution (e.g., 0.9% (w/v) NaCl), glucose solution (e.g., 5% glucose), solutions containing surfactants (e.g., 0.01% polysorbate 20), pH buffer solutions (e.g., phosphate buffer solutions), Ringer's solution, and any combination thereof.

As used in the present invention, the term "prevention" refers to a method implemented to block or delay the occurrence of a disease, condition, or symptom (e.g., a disease related to Ly6G6D) in a subject. As used herein, the term "treatment" refers to a method implemented to obtain beneficial or desired clinical results. For the purposes of the present invention, beneficial or desired clinical results include, but are not limited to, alleviation of symptoms, reduction in the scope of the disease, stabilization (i.e., no further deterioration) of the disease state, delay or slowing of disease progression, improvement or alleviation of the disease state, and remission of symptoms (whether partial or complete), whether detectable or undetectable. Furthermore, "treatment" may also refer to extending the survival period compared to the expected survival period if left untreated.

As used in the present invention, the term "subject" refers to a mammal, such as a primate mammal, for example, a human. In some embodiments, the subject (e.g., a human) suffers from a disease related to Ly6G6D.

The embodiments of the present invention will be described in detail below in conjunction with the accompanying drawings and examples. However, those skilled in the art will understand that the following drawings and examples are for illustrative purposes only and do not limit the scope of the present invention. Various objects and advantageous aspects of the present invention will become apparent to those skilled in the art from the following detailed description of the accompanying drawings and preferred embodiments.

### Brief Description of the Drawings

Figure 1 is a diagram showing the results of confirming the binding of antibodies to the hLY6G6D antigen by ELISA;
Figure 2 is a diagram showing the results of confirming the binding of antibodies to the cynoLY6G6D antigen by ELISA;
Figure 3 is a diagram showing the results of detecting the binding of antibodies to HEK23T cells highly expressing hLY6G6D by flow cytometry;
Figure 4 is a diagram showing the results of detecting the binding of antibodies to HEK23T cells highly expressing cynoLY6G6D by flow cytometry;
Figure 5 is a diagram showing the results of evaluating in vitro ADCC activity using the Jurkat-NFAT-Luc2-CD16 cell model.

### Detailed Description of Embodiments

The present invention will now be described with reference to the following examples, which are intended to illustrate the invention and not to limit it.

Unless otherwise specified, the molecular biology experimental methods and immunoassays used in the present invention are basically performed according to the methods described in J.Sambrook et al., Molecular Cloning: A Laboratory Manual, 2 nd Edition, Cold Spring Harbor Laboratory Press, 1989, and F .M .Ausubel et al., Current Protocols in Molecular Biology, 3 rd Edition, John Wiley&Sons ,Inc ., 1995. Those skilled in the art will appreciate that the examples describe the invention by way of illustration and are not intended to limit the scope of the claimed invention.

### Example 1: Screening of LY6G6D Nanobodies Using Phage Display Technology and Flow Cytometry

Nanobodies in the VH format were displayed on phages, and panning was performed using phage library screening technology. The ELISA method was employed to screen for monoclonal antibodies capable of high-level binding to Human LY6G6D. Genes encoding the extracellular domains of Human LY6G6D and cyno LY6G6D proteins were designed using the Uniprot and NCBI databases and constructed into a mammalian eukaryotic expression system to express the extracellular domains of Human LY6G6D and cyno LY6G6D proteins, which were used for immunization and nanobody library screening. Alpacas were immunized with the expressed Human LY6G6D extracellular domain protein six times until the titer reached 10⁴. PBMCs were isolated from alpaca blood, and VH sequences of nanobodies were amplified therefrom to establish an immune nanobody phage library. The immune nanobody phage library was panned against the Human LY6G6D protein. After three rounds of panning, the ELISA method was used to screen for monoclonal phages capable of binding to Human LY6G6D. The binding results of four candidate phages to Human LY6G6D are shown in Table 1 below:

**Table 1**

| Number | 1A4 | 1D5 | 1D5G8 | 2G5 |
|---|---|---|---|---|
| OD450 | 1.312 | 0.613 | 1.558 | 1.429 |

### Example2: Expression and Purification of Antibodies

The four candidate phages obtained in Example 1 were sequenced to obtain the heavy chain variable region sequences encoding the antibody candidates. Full gene synthesis was used to synthesize the gene coding sequences for heavy chain IgG antibodies (VH-Linker-Fc, wherein VH has the amino acid sequence shown in any one of SEQ ID NOs: 28-31, Fc has the amino acid sequence shown in SEQ ID NO: 32, and Linker has the amino acid sequence shown in SEQ ID NO: 33). A series of antibody expression vectors were constructed using molecular cloning methods and recombinantly expressed in the 293F expression system. 293F host cells were recovered and cultured in OPM medium. When the cell density reached approximately 3*10⁶ cells/mL, the cells were harvested and transfected using PEI reagent. On the second day of culture, feed and glucose were added to achieve a culture concentration of 8 g/L. On the sixth day of culture, the cell culture supernatant was collected.

A series of candidate antibodies were detected at the translation level. The collected cell culture supernatant was purified using a Protein A chromatography column, and the absorption peaks were collected for mass spectrometry analysis. Mass spectrometry analysis of the series of candidate antibodies showed that the molecular weight of the candidate antibody VHH-Fc was 75KDa, consistent with the theoretical molecular weight, indicating a dimeric form. Meanwhile, the collected samples were analyzed by 10% SDS-PAGE electrophoresis under both reduced and non-reduced conditions. The reduced SDS-PAGE electropherogram of the candidate antibody VH-Fc showed a band around 37KDa, while the non-reduced SDS-PAGE electropherogram showed a single band around 75KDa. The band sizes on the electropherograms were consistent with the theoretical values. The candidate antibody VHH-Fc, possessing a smaller size, exhibits superior tumor penetration capability. The purified samples were dialyzed overnight at 4°C against 0.02M PBS buffer at pH7.4.

### Example 3: Detection of Binding Characteristics Between Antibody and Antigen

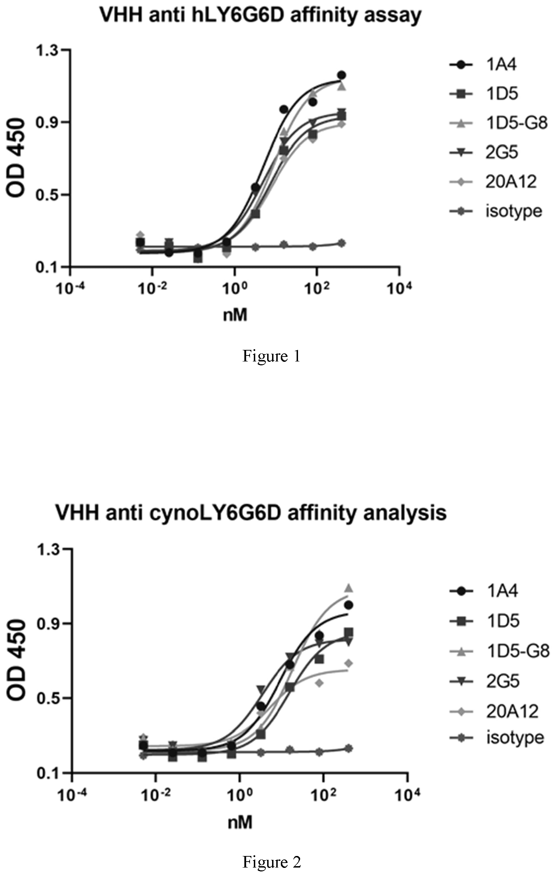

The binding of the expressed antibodies to the hLY6G6D antigen was confirmed by ELISA. The positive control antibody 20A12 was sourced from patent CN202080086360.1. ELISA plates were coated with 100 ng/well of hLY6G6D and incubated in an electric thermostatic incubator at 37°C for 2 h. Non-specific binding sites were then blocked with 5% skim milk powder at 4°C overnight. The plates were incubated with VHH at different concentrations for 1 h, washed three times with 0.1% PBST, and then incubated with HRP-mouse anti-human IgG1 Fc (SouthernBiotech) to detect the bound VHH. Subsequently, the plates were washed five times with 0.1% PBST, TMB substrate (Yunqiao Bio) was added, the reaction was stopped after color development, and absorbance was read at 450 nm. As shown in Figure 1 and Table 2, the affinity of the candidate antibodies was comparable to that of the positive control antibody 20A12, with the affinities of 1A4 and 2G5 being superior to that of 20A12. The heavy chain of the positive control antibody 20A12 comprises the amino acid sequence shown in SEQ ID NO: 34, and the light chain comprises the amino acid sequence shown in SEQ ID NO: 35.

EVQLLESGGGLVQPGGSLRLSCAASGFDFVNNAMIWVRQAPGKGLEWVSALSFADNT
AYYATWASGRFTISRDSSKTTVYLQMNSLRAEDTAVYYCMRGDLWGPGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPK
DTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSREEMTKNQVSLTCLV
KGFYPSDIAVEWESNGQPENNYKTTPPVLDSDGSFFLYSKLTVDKSRWQQGNVFSCSVMHE
ALHNHYTQKSLSLSPGK (SEQ ID NO: 34);

DIQMTQSPSTLSASVGDRVTITCQASESITRYLNWYQQKPGKAPKLLIYDASKLPSGVP
SRFSGSGSGTEFTLTISSLQPDDFATYYCQSTSFRGRSYQNTFGGGTKVEIKRTVAAPSVFIFP
PSDEQLKSGTASVVCLLNNFYPREAKVQWKVDNALQSGNSQESVTEQDSKDSTYSLSSTLT
LSKADYEKHKVYACEVTHQGLSSPVTKSFNRGEC (SEQ ID NO: 35).

**Table 2**

| No. | 1A4 | 1D5 | 1D5G8 | 2G5 | 20A12 |
|---|---|---|---|---|---|
| EC50( nM) | 5.03 | 7.329 | 8.756 | 4.917 | 7.89 |

### Example 4: Detection of Binding Characteristics Between Antibody and Antigen

Cross-reactivity assay with cynomolgus monkey: ELISA was used to confirm the binding of the expressed antibodies to the cynoLY6G6D antigen. The positive control antibody 20A12 is derived from patent CN202080086360.1. ELISA plates were coated with 100 ng/well of cynoLY6G6D and incubated in an electric thermostatic incubator at 37°C for 2 h. Non-specific binding sites were then blocked overnight with 5% skim milk powder at 4°C. The plates were incubated with VHH at various concentrations for 1 h, washed three times with 0.1% PBST, and detected with HRP-mouse anti-human IgG1 Fc (SouthernBiotech) to measure bound VHH. After washing five times with 0.1% PBST, TMB substrate (Yunqiao Bio) was added. The reaction was stopped after color development, and absorbance was read at 450 nm. As shown in Figure 2 and Table 3, both the candidate antibodies and the positive control antibody exhibited binding to cynoLY6G6D.

**Table 3**

| No. | 1A4 | 1D5 | 1D5G8 | 2G5 | 20A12 |
|---|---|---|---|---|---|
| EC50 (nM) | 9.275 | 15.33 | 18.47 | 3.473 | 5.239 |

### Example 5: Assay for Antibody-Antigen Binding Characteristics

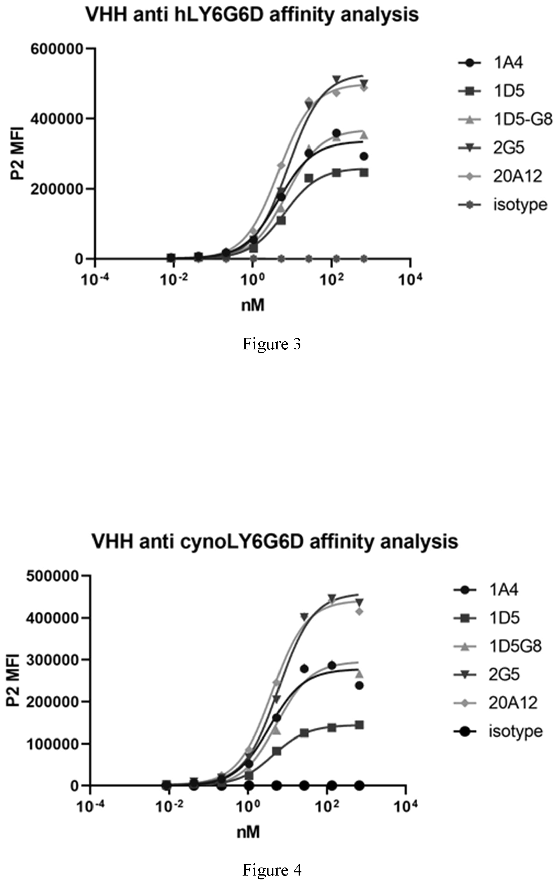

Flow cytometry was used to assess the binding of antibodies to HEK293T cells overexpressing hLY6G6D. The positive control antibody 20A12 is derived from patent CN202080086360.1. Cells (2×10⁵) were resuspended in a 96-well plate and incubated with VHH at various concentrations for 1 h. The cells were then washed twice with PBS. PE-conjugated Goat anti-human IgG Fc (Jackson ImmunoResearch), diluted 1:500, was added and incubated at 4°C for 30 min to detect bound antibodies. Subsequently, the cells were washed twice with PBS and analyzed on a FACS instrument (Beckman). As shown in Figure 3 and Table 4, the affinity of the candidate antibodies was comparable to that of the positive control antibody 20A12.

**Table 4**

| No. | 1A4 | 1D5 | 1D5G8 | 2G5 | 20A12 |
|---|---|---|---|---|---|
| EC50 (nM) | 4.569 | 6.313 | 7.137 | 7.938 | 4.565 |

### Example 6: Assay for Antibody-Antigen Binding Characteristics

Cross-reactivity assay with cynomolgus monkey: Flow cytometry was used to assess the binding of antibodies to HEK293T cells overexpressing cynoLY6G6D. The positive control antibody 20A12 is derived from patent CN202080086360.1. Cells (2×10⁵) were resuspended in a 96-well plate and incubated with VHH at various concentrations for 1 h. The cells were then washed twice with PBS. PE-conjugated Goat anti-human IgG Fc (Jackson ImmunoResearch), diluted 1:500, was added and incubated at 4°C for 30 min to detect bound antibodies. Subsequently, the cells were washed twice with PBS and analyzed on a FACS instrument (Beckman). As shown in Figure 4 and Table 5, both the candidate antibodies and the positive control antibody exhibited binding to cynoLY6G6D.

**Table 5**

| No. | 1A4 | 1D5 | 1D5G8 | 2G5 | 20A12 |
|---|---|---|---|---|---|
| EC50 (nM) | 3.483 | 4.309 | 5.299 | 5.957 | 4.072 |

### Example 7: Evaluation of In Vitro ADCC Activity Using the Jurkat-NFAT-Luc2-CD16 Cell Model

A Jurkat-NFAT-Luc2-CD16 reporter gene cell model was constructed using Jurkat cells stably transfected with the FcγRIIIa receptor and NFAT (nuclear factor of activated T cells) to evaluate the ADCC activity of candidate molecules.

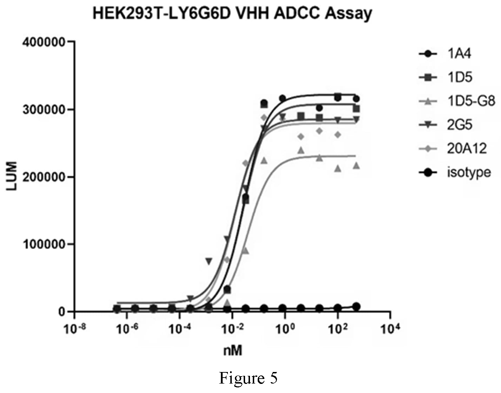

Using the constructed hLY6G6D-HEK293T cells as target cells and Jurkat-NFAT-Luc2-CD16 cells as effector cells, the effector and target cells were seeded in a 384-well plate at 5,000 cells per well. The positive control antibody 20A12 is derived from patent CN202080086360.1. Antibody dilutions at various concentrations were added to the 384-well plate. The effector and target cells were co-cultured in a 37°C, 5% CO₂ incubator for 14 h. The antibodies specifically bind to hLY6G6D on the surface of the target cells, and the Fc region of the antibodies binds to the FcγRIIIa receptors on the surface of the effector cells, thereby forming a target cell-antibody-effector cell complex. This activates the NFAT-Luc signaling pathway within the effector cells, leading to luciferase production. Upon addition of the luciferase detection substrate and a 3-min reaction, the luminescence value of each well was read using a multifunctional microplate reader. The results are shown in Figure 5 and Table 6; the ADCC activity of the candidate antibodies is comparable to that of the positive control antibody 20A12.

**Table 6**

| No. | 1A4 | 1D5 | 1D5G8 | 2G5 | 20A12 |
|---|---|---|---|---|---|
| EC50 (nM) | 0.02771 | 0.02655 | 0.03815 | 0.01326 | 0.01213 |

Although specific embodiments of the present invention have been described in detail, those skilled in the art will understand that various modifications and changes to the details can be made based on all the teachings disclosed herein, and such changes fall within the scope of protection of the present invention. The full scope of the present invention is defined by the appended claims and any equivalents thereof.

## Claims

1. A nanobody specifically binding to Ly6G6D, wherein the nanobody is capable of specifically binding to Ly6G6D, and a heavy chain variable region of the nanobody comprises complementarity determining regions: CDR1, CDR2 and CDR3;
wherein, the CDR1 has the amino acid sequence shown in SEQ ID NO: 1 or SEQ ID NO: 2;
YYAIG( SEQ ID NO: 1);
SYX₁ MX₂(SEQ ID NO: 2); wherein, X₁ is selected from A or M, and X₂ is selected from E or G;
the CDR2 has the amino acid sequence shown in SEQ ID NO: 3 or SEQ ID NO: 4;
CISSSGGSTVYADTVKG( SEQ ID NO: 3);
X₃X₄SWX₅GGX₆TYYADSX₇KG (SEQ ID NO: 4); wherein, X₃ is selected from V or A; X₄ is selected from I, H or V; X₅ is selected from T or Y; X₆ is selected from V or S; X₇ is selected from V or A;
the CDR3 has the amino acid sequence shown in SEQ ID NO: 5, SEQ ID NO: 6 or SEQ ID NO: 7;
GPECSRWYKDS( SEQ ID NO: 5);
ESRGFTHYNGNYYSRPDY( SEQ ID NO: 6);
RAPVKGDITPSNYYGLDY( SEQ ID NO: 7).

2. The nanobody according to claim 1, wherein the
CDR1 has the amino acid sequence shown in SEQ ID NO: 1, SEQ ID NO: 8, SEQ ID NO: 9 or SEQ ID NO: 10;
CDR2 has the amino acid sequence shown in SEQ ID NO: 3, SEQ ID NO: 11, SEQ ID NO: 12 or SEQ ID NO: 13;
CDR3 has the amino acid sequence shown in SEQ ID NO: 5, SEQ ID NO: 6 or SEQ ID NO: 7.

3. The nanobody according to claim 1 or 2, wherein the heavy chain variable region further comprises framework regions: FR1, FR2, FR3 and FR4; wherein, the framework regions and the complementarity determining regions are arranged alternately in sequence.

4. The nanobody according to claim 3, wherein,
FR1 has the amino acid sequence shown in SEQ ID NO: 14;
QX₈QLVESGGGLVQX₉GGSLRLSCAASGX₁₀TX₁₁X₁₂ (SEQ ID NO: 14); wherein, X₈ is selected from V or L; X₉ is selected from P or A; X₁₀ is selected from F or R; X₁₁ is selected from L or F; X₁₂ is selected from D, E or S;
FR2 has the amino acid sequence shown in SEQ ID NO: 15;
WFRQAPGKEREX₁₃VX₁₄ (SEQ ID NO: 15); wherein, X₁₃ is selected from G or F; X₁₄ is selected from S or A;
FR3 has the amino acid sequence shown in SEQ ID NO: 16;
RFTISRDNX₁₅KNX₁₆ X₁₇YLQMX₁₈SLKPEDTAVYYCAA (SEQ ID NO: 16); wherein, X₁₅ is selected from T or A; X₁₆ is selected from T or A; X₁₇ is selected from V or L; X₁₈ is selected from N or D;
FR4 has the amino acid sequence shown in SEQ ID NO: 17 or SEQ ID NO: 18;
WGQGTQVTVSS ( SEQ ID NO: 17);
SGKGTLVIVSS (SEQ ID NO: 18).

5. The nanobody according to claim 4, wherein the
FR1 has the amino acid sequence shown in SEQ ID NO: 19, SEQ ID NO: 20, SEQ ID NO: 21 or SEQ ID NO: 22;
FR2 has the amino acid sequence shown in SEQ ID NO: 23 or SEQ ID NO: 24;
FR3 has the amino acid sequence shown in SEQ ID NO: 25, SEQ ID NO: 26 or SEQ ID NO: 27;
FR4 has the amino acid sequence shown in SEQ ID NO: 17 or SEQ ID NO: 18.

6. The nanobody according to any one of claims 1-5, wherein the nanobody heavy chain variable region has the amino acid sequence shown in any one of SEQ ID NO: 28-31, or an amino acid sequence having at least 85%, at least 90%, at least 95% or at least 99% identity to any one of SEQ ID NO: 28-31.

7. An anti-Ly6G6D antibody, wherein the antibody is a conventional antibody or a functional fragment thereof, wherein a heavy chain variable region of the conventional antibody or functional fragment thereof is constituted by a nanobody specifically binding to Ly6G6D according to any one of claims 1-6.

8. The anti-Ly6G6D antibody according to claim 7, wherein the antibody functional fragment is a Fab, Fab', (Fab')2, Fv, scFv or sdFv structure of a conventional antibody.

9. A fusion protein, wherein the fusion protein comprises a functional domain capable of specifically binding to Ly6G6D; wherein the functional domain is constituted by a nanobody specifically binding to Ly6G6D according to any one of claims 1-6.

10. An isolated nucleic acid molecule, wherein the nucleic acid molecule comprises a nucleotide sequence encoding the nanobody according to any one of claims 1-6, the antibody according to claim 7 or 8, or the fusion protein according to claim 9;
preferably, the nucleic acid molecule is DNA.

11. An expression vector, wherein the expression vector carries the nucleic acid molecule according to claim 10;
preferably, the expression vector is a eukaryotic expression vector.

12. A host cell, wherein the host cell comprises the nucleic acid molecule according to claim 10 or the expression vector according to claim 11;
preferably, the host cell is a eukaryotic host cell;
more preferably, the host cell is a mammalian host cell.

13. A pharmaceutical composition, wherein the pharmaceutical composition comprises the nanobody according to any one of claims 1-6, the antibody according to claim 7 or 8, the fusion protein according to claim 9, the nucleic acid molecule according to claim 10, the expression vector according to claim 11, or the host cell according to claim 12;
preferably, the pharmaceutical composition further comprises a pharmaceutically acceptable carrier.

14. Use of the nanobody according to any one of claims 1-6, the antibody according to claim 7 or 8, the fusion protein according to claim 9, the nucleic acid molecule according to claim 10, the expression vector according to claim 11, the host cell according to claim 12, or the composition according to claim 13 in the manufacture of a medicament for treating or alleviating Ly6G6D-positive cancer in a subject in need thereof.

15. The use according to claim14, wherein the Ly6G6D-positive cancer is colorectal cancer, head and neck cancer, ovarian cancer, cervical cancer, melanoma, gastric cancer, esophageal cancer, small intestine cancer, large intestine cancer or adenocarcinoma.

16. A kit for detecting Ly6G6D, wherein the kit comprises the nanobody according to any one of claims 1-6, the antibody according to claim 7 or 8, the fusion protein according to claim 9, and instructions for use.
